(19) Europäisches Patentamt
European Patent Office
Office européen des brevets

(11) Numéro de publication: **0 385 835 A1**

# (12) DEMANDE DE BREVET EUROPEEN

(21) Numéro de dépôt: **90400510.5**

(51) Int. Cl.[5]: **C07C 209/10, C07C 211/52**

(22) Date de dépôt: **23.02.90**

(30) Priorité: **03.03.89 FR 8902755**

(43) Date de publication de la demande:
**05.09.90 Bulletin 90/36**

(84) Etats contractants désignés:
**AT BE CH DE DK ES FR GB GR IT LI LU NL SE**

(71) Demandeur: **RHONE POULENC CHIMIE**
**25 Quai Paul Doumer**
**F-92408 Courbevoie Cédex(FR)**

(72) Inventeur: **Béziat,Yves**
**799 avenue du Miradou**
**F-34 Saint Clément Lazrivière(FR)**
Inventeur: **Cristau,Henri-Jean**
**3 Le Clos du Village**
**F-34 Saint-Aunes(FR)**
Inventeur: **Desmurs,Jean-Roger**
**La Jonquière Route de Ternay**
**F-69360 Communay(FR)**
Inventeur: **Ratton,Serge**
**Vaulx-Milieu**
**F-38290 La Verpillière(FR)**

(74) Mandataire: **Ricalens, François et al**
**RHONE-POULENC CHIMIE Service Brevets**
**Chimie 25, quai Paul Doumer**
**F-92408 Coubevoie(FR)**

## (54) Procédé de préparation de n-allylmetatrifluoromethylaniline.

(57) La présente invention concerne un procédé de préparation de la N-monoallylmétatrifluorométhylaniline qui consiste à condenser le métabromotrifluorométhylbenzène avec l'allylamine en présence d'un catalyseur à base de nickel à l'état d'oxydation 2.

EP 0 385 835 A1

## PROCEDE DE PREPARATION DE N-ALLYLMETATRIFLUOROMETHYLANILINE

La présente invention concerne un nouveau procédé de préparation de la N-monoallylmétatrifluorométhylaniline.

La préparation du dérivé monoallylé de la métatrifluorométhylaniline est essentielle car il est un intermédiaire important dans la synthèse d'un herbicide tel que décrit dans le brevet FR 2305434. Selon ce brevet, pour préparer l'herbicide désiré, la N-métatri fluorométhylphényl-1 chloro-3 chlorométhyl-4 pyrrolidone-2, il est nécessaire de partir d'une trifluorométhylaniline dont un des atomes d'hydrogène est protégé par un groupe acétyle avant de procéder à l'allylation, ceci afin d'éviter la formation de produits secondaires de diallylation qui sont inutilisables.

L'industrie cherche depuis longtemps à accéder à la N-monoallyltrifluorométhylaniline directement en une seule étape, au lieu des trois étapes décrites dans le brevet FR 2305434, avec de bons rendements calculés sur la matière première engagée la métatrifluorométhylaniline qui est un composé très onéreux que l'industrie ne veut pas perdre.

Une première solution à ce problème a été proposée dans le brevet US 4701560 qui décrit un procédé d'allylation de la métatrifluorométhylaniline en milieu biphasique, eau-solvant organique en présence d'une base minérale choisie parmi les carbonates ou la soude et en présence de quantités catalytiques d'une amine tertiaire quaternisable. Pour obtenir une faible quantité de produits secondaires diallyliques, il est nécessaire de limiter le taux de transformation de la métatrifluorométhylaniline et donc de travailler en présence d'un défaut d'halogénure d'allyle, il est précisé dans ce texte que le rapport métatrifluorométhylaniline à l'halogénure d'allyle est de préférence d'environ 2. Les rendements en N-monoallylaniline calculés sur la métatrifluoro méthylaniline introduite ne dépasse pas 40 % ce qui est insuffisant pour une bonne rentabilité économique du procédé.

Malgré l'existence d'une abondante littérature décrivant l'alkylation ou l'allylation de diverses anilines, aucun procédé n'a jamais permis de résoudre le problème évoqué dans la présente invention c'est-à-dire avoir un bon taux de transformation de l'aniline de départ et obtenir une bonne sélectivité aniline monosubstituée sur l'azote par rapport à l'aniline disubstituée.

La présente invention a permis d'atteindre cet objectif.

Elle concerne un procédé de préparation de N-monoallylméta trifluorométhylaniline caractérisé en ce que l'on condense sur le métabromotrifluorométhylbenzène l'allylamine en présence d'un catalyseur à base d'un sel de nickel à l'état d'oxydation 2 et d'un ligand.

La réaction peut être schématisée par l'équation suivante :

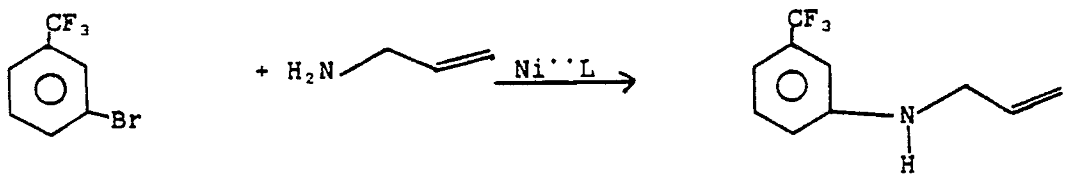


Il n'était pas évident que la présence concommitante du nickel et d'un réactif comportant un groupement allyle puisse permettre à la réaction d'évoluer. En effet il est connu d'après l'article de WILKE, BOGDANOVIC, HARDT, HEIMBACH, KEIM, KRÖNER, OBERKIRCH, TANAKA, STEINRÜCKE, WALTER, ZIMMERMANN (Angewandte chemie, Intern. Edition, 1966, 151-266) que les réactifs comportant un groupement allyle et le nickel forment des complexes piallyliques relativement stables. On pouvait donc s'attendre à ce que le nickel soit complexé et à ce que la réaction s'arrête à ce stade intermédiaire. Or il a été remarqué avec surprise que la réaction n'était pas gênée par la formation de cet intermédiaire mais évoluait favorablement, spécifiquement vers la formation d'une mono allylamine aromatique en l'absence de tout autre composé de condensation, par exemple directement sur le noyau aromatique ailleurs qu'en méta par rapport au groupe trifluorométhyle.

Le nickel à l'état d'oxydation 2 est choisi par exemple parmi

- le dichlorure de nickel
- le dibromure de nickel
- le sulfate de nickel
- le dinitrate de nickel
- le diacétate de.nickel
- le carbonate de nickel

- le dibenzoate de nickel
- le dihydroxyde de nickel
- le nickelocène (ou dicyclopentadienyl de nickel)
- l'oxyde de nickel

On préfère parmi l'ensemble des sels de nickel utiliser le dibromure.

Le ligand L est choisi parmi les ligands hydrocarbonés soit azotés, soit phosphorés, soit oxygénés.

On peut citer à titre d'exemples parmi les ligands azotés :

- le bipyridyle
- la phénantroline
- les dérivés azotés de formule (I)

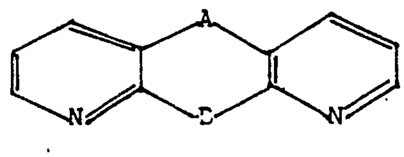

dans laquelle A et B représentent chacun un hétéroatome, une chaîne alkylidène, ou une liaison covalente

On peut citer parmi les ligands phosphorés :

les diverses phosphines telles que par exemple :

- l'orthophénylènediphosphine
- le bis 1,2-(diphénylphosphine) éthylène
- le bis 1,2-(diphénylphosphine) éthane

La réaction peut avoir lieu en présence d'un excès d'un réactif ou peut avoir lieu dans un solvant. Le solvant devra ne pas réagir avec les matières premières mises en oeuvre et devra préférentiellement solubiliser les réactifs.

On peut utiliser les hydrocarbures aromatiques ou aliphatiques tels que le diméthoxyéthane, le monoéther éthylique ou méthylique du diéthylène glycol, le diéther éthylique ou méthylique du diéthylène glycol, les éthers, le tétrahydrofurane, les alcools aliphatiques tels que l'éthanol, le propanol, le butanol.

Pour une meilleure mise en oeuvre de l'invention on préfère utiliser une quantité d'allylamine telle que le rapport molaire de l'allylamine au métabromotrifluorométhylbenzène soit supérieur à 1 et de préférence compris entre 1 et 10.

On préfère aussi utiliser une quantité de catalyseur telle que le rapport molaire du catalyseur ligandé au métabromotrifluorométhyl benzène soit compris entre 0,005 et 0,15.

La réaction est de préférence mise en oeuvre à une température comprise entre la température ambiante et 250 °C et de préférence entre 100 et 200 °C.

La pression utilisée sera généralement la pression autogène créée par le milieu réactionnel à la température de réaction.

L'invention sera plus complètement décrite à l'aide des exemples suivants qui ne doivent pas être considérés comme limitatifs de l'invention.

<u>EXAMPLE 1</u> Synthèse du catalyseur

Synthèse du dibromobis(O-phénanthroline)nickel II ou $(Phen)_2NiBr_2$

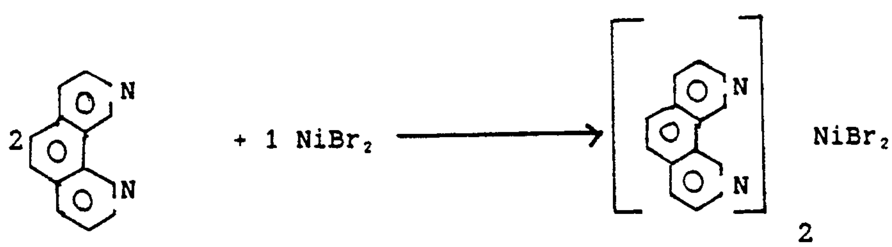

Mode opératoire :

A une suspension de 0,74 g (3,4 m mol) de $NiBr_2$ anhydre dans 70 $cm^3$ d'éthanol à 40 °C, on ajoute une solution de 1,35 g (6,8 m mol) d'orthophénanthroline dans 100 $cm^3$ d'éthanol.

Une coloration verte apparaît rapidement.

Le mélange réactionnel est porté au reflux pendant 3 heures. Le précipité vert formé est filtré et séché à 80 °C, sur $P_2O_5$, sous 0,5 torr pendant 18 h.

On récupère 1,48 g (2,6 m mol ; Rdt = 76 %) d'un solide vert.

<u>EXEMPLE 2</u> Synthèse du catalyseur

Synthèse du dibromobis(bipyridine)nickel II = $(Bpy)_2NiBr_2$

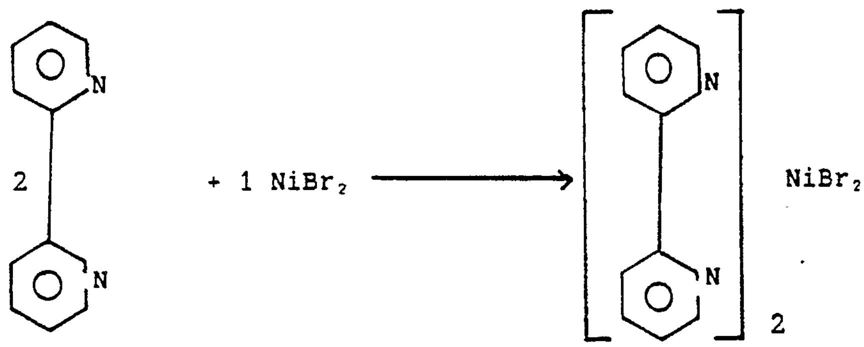


Mode opératoire :

A une suspension à 40 °C de 0,78 g (3,57 m moi) de $NiBr_2$ anhydre dans 70 $cm^3$ d'éthanol, on ajoute une solution de 1,11 g (7,14 m mol) de bipyridine dans 10 $cm^3$ d'éthanol.

Une coloration verte apparaît immédiatement.

Le mélange réactionnel est porté au reflux pendant 15 heures. La solution est alors filtrée à chaud. Le produit vert obtenu par refroidissement du filtrat concentré est lavé à l'éthanol et séché à 70 °C, sur $P_2O_5$, sous 0,5 torrs pendant 24 h.

On récupère 1,5 g (2,8 m mol ; Rdt = 78,1 %) d'un solide vert.

<u>EXEMPLES 3 A 7</u> Synthèse de la N-monoallylmétatrifluorométhylaniline

Les réactions ont été réalisées dans des tubes en verre, scellés, pouvant résister à une pression d'environ 50 bar.

Le catalyseur solide est pesé puis introduit par un entonnoir dans le tube. Les réactifs liquides sont introduits par volume à l'aide d'une pipette.

Le tube est refroidi à - 40 °C dans l'éthanol puis scellé.

Le tube scellé est ensuite introduit dans un autoclave en acier qui est placé dans une gaine chauffée et agitée.

A la fin de la réaction le tube est refroidi puis descellé.

Le contenu du tube est additionné de 15 ml de soude 1N et extrait par 3x10 ml d'éther.

La phase organique est filtrée sur verre fritté et célité puis analysée en C.P.G.

Un essai comparatif a été réalisé dans les mêmes conditions qu'à l'exemple 3 en utilisant un catalyseur à base de nickel à l'état d'oxydation zéro, le $Ni[P(C_6H_5)_3]_4$.

| Exemples | 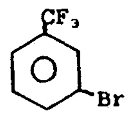 | 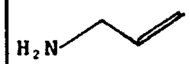 | Solvant | Catalyseur % molaire | Conditions | TT 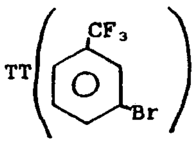 | RT 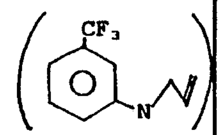 |
|---|---|---|---|---|---|---|---|
| 3 | 0,45 g | 1,15 g | Diméthyl éther 1,2 ml | $NiBr_2$ 3,5 % | 18h, 160° C | 19 % | 100 % |
| 4 | 0,45 g | 1,15 g | Diméthyl éther 1,2 ml | $NiBr_2$ 11 % | 18h, 160° C | 23 % | 100 % |
| 5 | 0,45 g | 1,15 g | Diméthyl éther 1,2 ml | Ex 1 11 % | 18h, 160° C | 30 % | 100 % |
| 6 | 0,45 g | 1,15 g | Diméthyl éther 1,2 ml | Ex 2 11 % | 18h, 160° C | 45 % | 100 % |
| 7 | 0,45 g | 1,15 g | Ethanol 1,2 ml | Ex 2 3,5 % | 18h, 160° C | 82 % | 84 % |
| COMPARATIF | 0,45 g | 1,15 g | Diméthyl éther 1,2 ml | $Ni[P(C_6H_5)_3]_4$ 3,5 % | 18h, 160° C | 9 % | 45 % |

EXEMPLES 8 A 10 Synthèse du catalyseur le dibromo bis 1,2-(diphénylphosphine) éthylène nickel

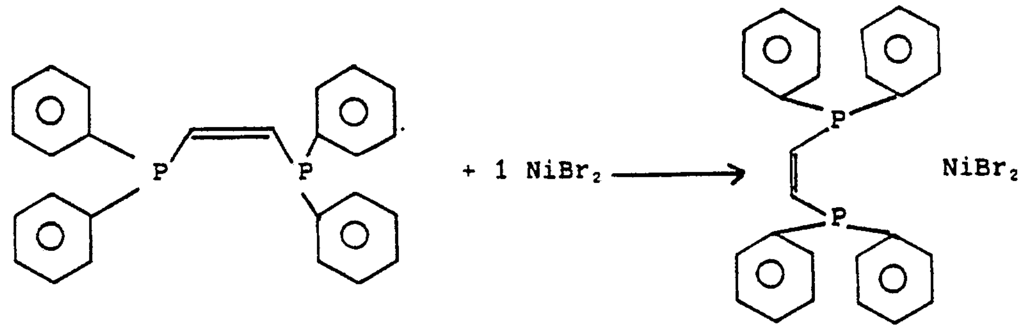

0,55 g de $NiBr_2$, $3H_2O$ sont introduits dans 1 ml d'éthanol. On mélange 0,8 g d'éthylènediphosphine dissous dans 10 ml d'éthanol et ml de toluène. On chauffe au reflux et on ajoute le bromure de nickel. Il se forme un précipité brun du complexe éthylène diphosphine $NiBr_2$. Le rendement de la synthèse atteint 88 %.

Le catalyseur est isolé et 0,07 millimoles sont introduites dans un tube scellé, comme à l'exemple 3, en présence de 0,002 mole de métatrifluorométhylbromobenzène, de 0,020 mole d'allylamine et de 1,2 ml d'éthanol. On chauffe à 160° C pendant 12 heures.

Dans l'exemple 9, on reproduit l'exemple 8 en synthétisant le catalyseur dans le tube en verre scellé directement. Les conditions de réaction sont identiques à celles de l'exemple 8.

| Exemples | 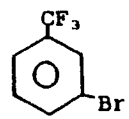 | 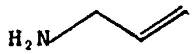 | Solvant | Catalyseur | Conditions | TT 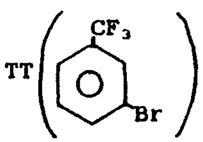 | RT 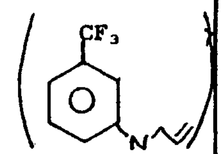 |
|---|---|---|---|---|---|---|---|
| 8 | 0,45 g | 1,15 g | Ethanol 1,2 ml | Ethylènediphos-phine $NiBr_2$ 3,5 % | 12h, 160° C | 34 % | 50 % |
| 9 | 0,45 g | 1,15 g | Ethanol 1,2 ml | Ethylènediphos-phine $NiBr_2$ 3,5 % | 12h, 160° C | 35 % | 40 % |
| 10 | 0,45 g | 1,15 g | Ethanol 1,2 ml | $NiBr_2$ 3,5 % | 12h, 160° C | 19 % | 100 % |

## Revendications

1. Procédé de préparation de N-monoallylmétatrifluorométhylaniline caractérisé en ce que l'on condense sur le métabromotrifluoro méthylbenzène l'allylamine en présence d'un catalyseur à base de nickel à l'état d'oxydation 2.

2. Procédé selon la revendication 1 caractérisé en ce que le nickel à l'état d'oxydation 2 est le dibromure de nickel.

3. Procédé selon la revendication 1 caractérisé en ce qu'on ajoute un ligand du nickel.

4. Procédé selon la revendication 3 caractérisé en ce que ce ligand est le bipypridyl.

5. Procédé selon le revendication 1 caractérisé en ce que la condensation a lieu en présence d'un solvant choisi parmi le diméthoxyéthane, les monoéthers du diéthylène glycol, les diéthers du diéthylène glycol, les éthers, les alcools.

6. Procédé selon la revendication 1 caractérisé en ce que le rapport molaire de l'allylamine au métabromotrifluorométhyl benzène est supérieur à 1 et de préférence compris entre 1 et 10.

7. Procédé selon la revendication 1 caractérisé en ce que le rapport molaire du catalyseur ligandé au métabromotrifluoro méthylbenzène est compris entre 0,005 et 0,15.

8. Procédé selon la revendication 1 caractérisé en ce que la température réactionnelle est comprise entre 100 et 200 °C.

Office européen des brevets

# RAPPORT DE RECHERCHE EUROPEENNE

Numero de la demande

EP 90 40 0510

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | THE JOURNAL OF ORGANIC CHEMISTRY vol. 40, no. 16, 8 août 1975, pages 2267-2273, Easton, US; R. CRAMER et al.: "Nickel-Catalyzed Displacement Reactions of Aryl Halides" * pages 2267-2271; page 2269, colonne 2; page 2270, colonne 2, tableau VIII * | 1-3,5,8 | C 07 C 209/10<br>C 07 C 211/52 |
| Y | US-A-3 888 928 (DONALD WILLIS) * colonne 6, exemple 2 * | 1-3,5,8 | |
| A | US-A-3 914 311 (D.R. COULSON) * colonnes 1-4; revendications 1,2,4-7 * | 1-3,5-8 | |
| A | EP-A-0 205 391 (RHONE-POULENC SPECIALITES CHIMIQUES) * résumé *; & US - A - 47 01560 (Cat. D) | 1 | |

| DOMAINES TECHNIQUES RECHERCHES (Int. Cl.5) |
|---|
| C 07 C 209/00<br>C 07 C 211/00 |

Le présent rapport a été établi pour toutes les revendications

| Lieu de la recherche | Date d'achèvement de la recherche | Examinateur |
|---|---|---|
| BERLIN | 26-04-1990 | RUFET J.M.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant